# EUROPEAN PATENT SPECIFICATION

(11) **EP 2 895 071 B1**
(45) Date of publication and mention of the grant of the patent: **17.05.2017**
(21) Application number: 13762522.4
(22) Date of filing: 17.09.2013
(51) Int. Cl.: A61B 5/1486, A61B 5/1473, C12Q 1/00, G01N 27/327, C23C 16/06, C23C 14/16

(54) **ROD SHAPED IMPLANTABLE BIOSENSOR**
STABFÖRMIGER IMPLANTIERBARER BIOSENSOR
BIOCAPTEUR IMPLANTABLE EN FORME DE TIGE

(30) Priority: 17.09.2012 US 201261702098 P
(43) Date of publication of application: 22.07.2015
(73) Proprietor: Brains Online Holding B.V., 9747 AW Groningen (NL)
(72) Inventor: CREMERS, Thomas Ivo Franciscus Hubert, NL-9747 AW Groningen (NL)
(74) Representative: Ellens, Andries
(86) International application number: PCT/EP2013/069248
(87) International publication number: WO 2014/041190

(56) References cited:
- WO-A1-2009/053370
- WO-A2-2012/075331
- CN-A- 102 507 690
- YANG Q ET AL: "Development of needle-type glucose sensor with high selectivity", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 46, no. 3, 15 May 1998 (1998-05-15), pages 249-256, XP004147305, ISSN: 0925-4005, DOI: 10.1016/S0925-4005(98)00126-9
- Anonymous: "AFN - Animal Feeding Needles", , 9 February 2011 (2011-02-09), pages 1-2, XP055262881, USA Retrieved from the Internet: URL:http://web.archive.org/web/20110209192 124/http://www.feedingneedles.com/ [retrieved on 2016-04-05]

## Description

### FIELD OF THE INVENTION

The present disclosure relates generally to brain-implantable biosensors. Especially, the present disclosure relates (more) generally to a tissue-implantable biosensor. The invention further relates to a biosensor and to the use thereof. Further, the present invention relates to the use of such biosensor, amongst others for sensing tissue extracellular metabolites (endogenous analyte).

### BACKGROUND OF THE INVENTION

Electrode assemblies for in vivo or in vitro sensing are known in the art. US2011/295097, for instance, describes an electrode assembly comprising a fetal electrode that is connected to a drive tube by a torque limiting connection. The connection allows the drive tube to separate from the electrode hub once a predetermined torque has been reached. The electrode hub is also provided with a deflection surface that deflects the drive tube away from the fetal electrode into the hand of the operator, as rotation of the drive tube continues beyond the point of disconnection. Features are also provided to make the fetal electrode more compact and to optimize the fECG signal recorded on the electrode wires.

WO2009/053370 describes an electrode comprising a conducting substrate for detecting an electroactive agent, a catalytic agent held on the substrate for converting a non-electroactive agent to an electroactive agent by a catalytic process which consumes oxygen, and an oxygen reservoir, for releasing oxygen to feed the catalytic process, held within a polymer matrix on the substrate. The electrode allows for detection of materials such as glucose when using oxidase enzymes. WO2009/053370 mentions the problem of fluctuation or depletion of oxygen in the environment in which the measurements are being taken. The conducting substrate comprises a non-metallic conductor such as carbon fibres or metallic conductors including those constructed of noble metals such as Pt, Ag, Au, Ru, Rh, Pd, Re, Os, Ir and combinations thereof. Further, the conducting substrate is formed by a conducting material coated onto a support such as a support comprising non-conductive material.
The catalytic agent is selected from oxidase materials, such as selected from glucose oxidase, lactate oxidase and glutamate oxidase and combinations thereof.

WO2012/075331 describes a biosensor system comprising a capillary substrate, conductive electrode, and a plurality of nanoparticles having an enzyme deposited thereon formed in a cavity at one end of the capillary substrate. The substrate may have an optional reinforcing layer (which may be conductive or non-conductive) and optional insulating layer thereon. A cannula having an optional conductive layer, insulating layer, and reference electrode may also form part of the system.

### SUMMARY OF THE INVENTION

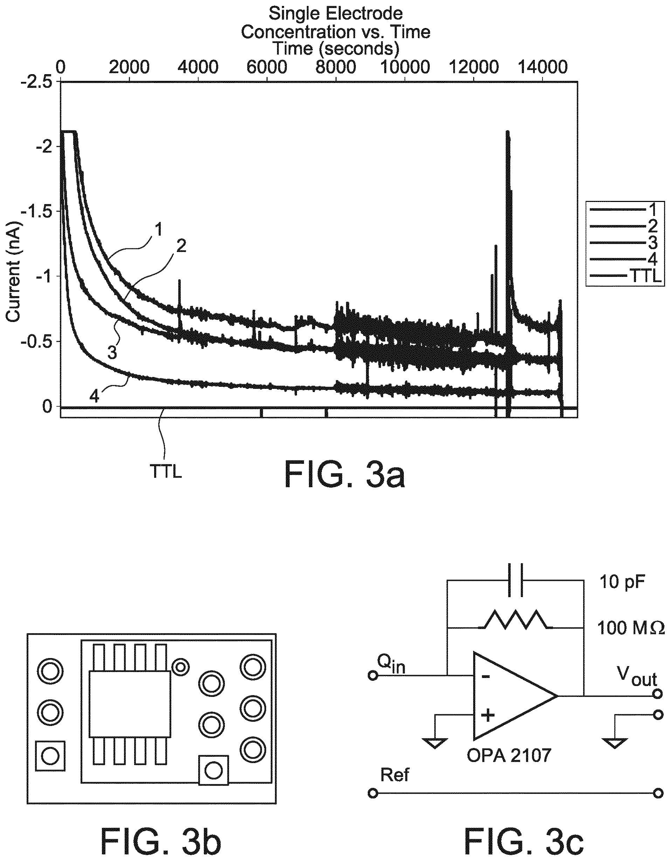

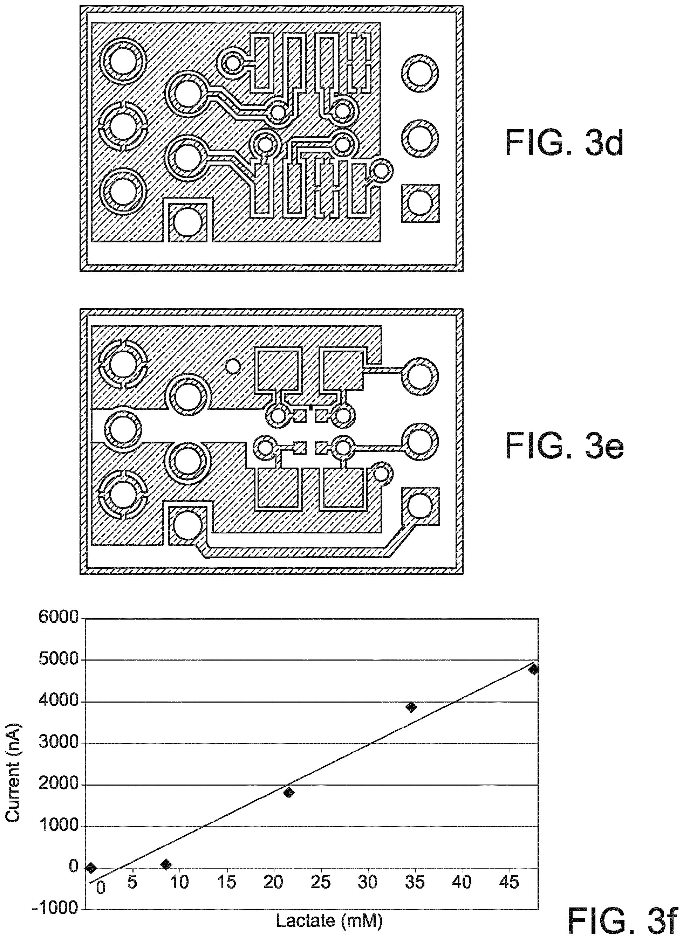

Several methods exist to monitor neurotransmitter levels in tissue, such as the brain and peripheral tissue of freely moving animals. Microdialysis and voltammetry are both accepted methods, but lack either temporal resolution or specificity for non-electrochemically active analytes. Typical sensors in vivo in tissue, such as brains of freely moving animals, generate low nA or high pA currents, which pushes sensor electronics and connections to their limit. Two problems may typically arise when sensors are applied in the tissue, such as the brain of freely moving animals: (1) Freely moving animals move and hence are susceptible to picking up noise due to movement (of electrical connections) and interference (cable noise) in electronics. Fig 3a shows typical change of noise when the activity state of animals changes during experiments; and/or (2) Downscaling. While sensors would ideally be the size of a neuron (< 10 micrometer), the fragility of sensor materials like carbon and platinum do not allow these dimensions. To this end sensors are typically >100 micrometer in diameter (for platinum wire sensors), or use a carrier for the sensors, which increases the size to >100 micrometer dimensions.

Hence, it is an aspect of the invention to provide an alternative biosensor and/or an alternative electrode for such biosensor, which preferably further at least partly obviate one or more of above-described drawbacks. It is further an aspect of the invention to provide an alternative sensing system, especially including such biosensor and/or electrode, which preferably further at least partly obviate one or more of above-described drawbacks.

Hence, in a first aspect the invention provides a biosensor comprising a biosensor unit with an electrode, wherein the electrode is rod-shaped, wherein the electrode further comprises a support, especially a core, with an electrically conductive first layer and an exclusion layer, wherein the electrically conductive first layer is configured between the support, especially the core, and the exclusion layer, as further defined in the accompanying claims. With such bionsensor, advantageously the central nerveus system may be monitored, for instance by monitoring neurotransmitters in tissue. The present biosensor allows rather thin electrodes with good strength, whereas prior art electrodes are often bulky and can e.g. not be used for sensing in tissue of a child, such as scalp tissue, during delivery. Surprisingly good and stable results were obtained, with these electrodes. However, the present biosensor also allows a large freedom of design, including thicker electrodes. The biosensor can be used for in vivo and/or in vitro sensing. For instance, the biosensor may use small implantable electrodes made of platinum or carbon, coated with enzymes that generate peroxide when in contact with certain analytes. For instance central levels of glutamate can be monitored when sensor electrodes are coated with glutamate oxidase enzymes. Peroxide (H₂O₂) is typically monitored using oxidation at 700 mV vs. Ag/AgCl reference electrodes. Typically, a potential difference between the electrode and a counter electrode is in the range of -1V - + 1V (vs. reference electrode)

The electrode has a rod-like shape, such as a needle. Especially rod having larger width, like > 100 µm may have a needle shape with a (sharp) tip. Hence, the electrode comprises a needle shape, especially the electrode has a needle shape tip. The rod may be configured to facilitate penetrating the skin, and optionally also subcutaneous tissue, and dependent upon the application also to facilitate (at least partial) penetrating of bone or skull. Especially, the electrode may be configured to facilitate (at least partial) penetration of soft tissue. However, in further aspects, the electrode has not necessarily a (substantially) round cross section. In even yet further aspects, the electrode may have a square or rectangular cross-section.

The electrode further comprises a support, such as a core, with an electrically conductive first layer and an exclusion layer, wherein the electrically conductive first layer is configured between the support, such as the core, and the exclusion layer. Hence, the electrode is rod shaped, with a (central core). The support, especially the core, may comprise a (hard) biocompatible material. For instance, this biocompatible material may comprise a steel, such as a stainless steel. However, other materials may also be possible. In specific variants, the support, especially the core, comprises a material having a compression strength of at least 20,000 kPa. Such hard material may especially be suitable for penetrating into a body of a human or animal. The support, especially the core, comprises a material selected from the group consisting of stainless steel, a carbide, titanium (Ti) (metal), vanadium (V) (metal), tantalum (Ta) (metal), and tungsten (W) metal. Alternatively or additionally, also one or more of graphene and carbyne may be possibe as support, especially core, material. It is also contemplated that rigid material other than tungsten can be used as the coated core, or alone, without coating. Such other materials include stainless steel or Ag₂O, for example. In addition, other coatings than platinum can be applied to such coatings, depending on the nature of the enzyme or other materials to be detected (see further below). Also alloys may be used (as support material, especially core material). However, other materials as support material are not excluded. For instance, metal matrix composites (such as one or more of tungsten carbide, silicon carbide, and chromium carbide, aluminium oxide), might also be applied. In an embodiment, also aluminium oxide may be applied. Hence, in an embodiment the support comprises a metal. In yet another embodiment the support comprises a metal alloy. In yet another embodiment, the support comprises a carbon based material, such as graphene and/or carbyne. In yet a further embodiment, the support comprises an oxide. The support is further defined in the accompanying claims.

Further, especially materials, even more especially metals or metal alloys, may be applied that have a shear modulus of at least 40 GPa, such as Cast Iron,
Phosphor Bronze, Titanium Grade 2, Titanium Grade 5, Titanium 10% Vanadium, Zinc, Copper, Beryllium Copper, Nickel Silver, Iron Malleable, Monel metal, Steel Cold-rolled, Nickel Steel, Z-nickel, Carbon Steel, Steel Cast, Inconel, Chromium, Molybdenum, Tungsten, Bronze, Iron Ductile, Stainless Steel, and Structural Steel. Especially, the support material, such as the core, comprises an electrically conductive material. This functionality can be used as the support may be part of the electrical system. However, not necessarily such functionality is applied. For instance, one can also use a support with two or more electrodes, with e.g. an insulator (such as Teflon) between the support and the electrodes. As indicated above, the support, especially the core, may comprises one or more biocompatible materials. This may be relevant in view of the penetration of tissue. Herein, the term "material" may also relate to a combination of materials, including alloys (see also above).

For measuring an analyte an active surface has to be created that allows measuring such analyte. To this end, the electrode may include an electrochemically active layer which may faciliate measuring the desired species at the desired potentials and/or currents. Further, to this end the electrode may include an exclusion layer, which facilitates that certain species do not reach the electrochemically active layer whereas others do. Further, especially the electrode may optionally include a specific enzyme that converts a biological molecule (analyte) to a species (such as a metabolite) that can be sensed by the biosensor. This will further elucidated below.The electrochemical quantification that occurs at the conductive layer is typical by amperometry, applying a fixed potential between an elctrode and a reference electrode.The current embodiments could also be used in coulometric and voltametric approaches.

In an embodiment, the electrically conductive first layer comprises one or more of gold (Au), silver (Ag), silver oxide (Ag₂O), platinum (Pt), and carbon (C), as further defined in the accompanying claims. For instance, Pt (platinum) may typically be applied as 0.5-5 µm layer and Au (gold) may typically be applied as 0.25-5 µm layer. Alternatively or additionally, one or more of the following materials may be applied: Cu (copper), Glassy carbon, Graphene, magic diamond, Rhodium, Chromium, Zinc, Cadmium, Nickel (such as electroless nickel), and Iridium. As indicated herein, also alloys of two more more metals may be applied. Especially, gold, silver and platinum are available as (useful) metal coating. The electrically conductive first layer (or herein also indicated as "electrically active layer" or "electrochemically active layer") can for instance be provided by chemical vapour deposition and/or sputtering. Other methods may e.g. include electro deposition or dipping (dip coating). The electrically conductive first layer can be configured directly on the (hard metal) support, such as the core, but there may also be one or more intermediate layers between the support and the electrically conductive first layer (see also below). In specific embodiments the electrically conductive first layer has a layer thickness in the range of 3 nm - 100 µm, such as 50 nm - 150 µm. Thinner or thicker layers may not lead or contribute to the desired signal (noise ratio) and/or selectivity. The electrically conductive first layer may include a multi layer, including a multilayer of different materials, like Au and Ag. The term "multi layer" especially indicates layers on top of each other.

As indicated above, there is further available an exclusion layer. In general, this exclusion layer is configured directly on the electrically conductive first layer, without intermediate layer. This may improve the selectivity of the sensor. The exclusion layer may also be indicated as "repelling layer". In a specific embodiment, the exclusion layer is permeable for one or more of H₂O₂, O₂, NO, and/or allows electron transfer, and wherein the exclusion layer is especially impermeable to one or more of ascorbate, 3,4-dihydroxyphenylacetic acid (DOPAC), dopamine, and uric acid. Herein, the term "permeable" may especially indicate that one or more of the indicates species are able to penetrate this layer. This penetration may be allowed due to the thickness of the layer and the type of material for the exclusion layer chosen. Such exclusion layer may block more bulky chemical molecules (including charged molecules), such as one or more of ascorbate, 3,4-dihydroxyphenylacetic acid (DOPAC), dopamine, and uric acid, but allow penetration of one or more of H₂O₂, O₂, NO (nitrogen oxide) and/or allow electron transfer. In this way, such smaller molecule may reach the electrically conductive first layer (i.e. especially the electrochemically active layer), and contribute to an electrical signal. In a specific embodiment, the exclusion layer is permeable for H₂O₂ (hydrogen peroxide). The difference in permeability between permeable and impermeable, such as e.g. measured in Barrer, between e.g. H₂O₂ and ascorbate can e.g. be defined as the permeability for H₂O₂ is at least 1,000 higher than the permeability for ascorbate. Hence, in an embodiment, the exclusion layer has a permeability for H₂O₂ that is at least 1,000, even more at least 10,000 higher than the permeability for ascorbate (at room temperature). Hence, the exclusion layer may be a selectively permeable layer

The exclusion layer may be provided by e.g. dip coating or other types of wet coating (of a starting material) with optionally a subsequent treatment like (electro)polymerization or curing to provide the coating of the desired material.

In a specific embodiment, the exclusion layer comprises one or more of sulfonated tetrafluoroethylene based fluoropolymer-copolymer, N,N'-Di-[(1-naphthyl)-N,N'-diphenyl]-1,1'-biphenyl)-4,4'-diamine (NPD), and p-Phenylenediamine (PPD). Especially, the exclusion layer comprises Nafion, a sulfonated tetrafluoroethylene based fluoropolymer-copolymer. Materials other than Nafion that may also be applied may include one or more of a polyphylene diamine, such as indictated above, (especially overoxidized) polypyrole, (especially overoxidized) polyaniline, and polynaphtol. Especially, the exclusion layer has a layer thickness in the range of 1 nm - 100 µm, especially at least 50 nm, such as 0.05-100 µm, like 1-50 µm. Thinner coatings may lead to undesired permeability and thus undesired influence of the electrical signal by molecules one does not necessarily desire to measure, whereas thicker coating may lead to loss of signal as not enough of the molecule of interest may penetrate the exclusion layer. The exclusion layer may include a multi layer, including a multilayer of different materials, like two or more of the above indicated polymers. As indicated above, also here the term "multi layer" especially indicates layers on top of each other.

In view of the fact that e.g. voltammetry or amperometry, etc., may be applied, it may be desireable to include in the electrode a layer that improves electrical conductivity. This may e.g. also depend whether or not the support can be used as electrical conductor. Hence, optionally an electrically conductive second layer is also comprised by the electrode. Whereas this layer may especially be configured to improve electrical conductivity, the electrically conductive first layer is especially a functional layer being electrochemical active with respect to the species to be measured (see also above). Hence, in a further embodiment the biosensor further comprises an electrically conductive second layer configured between the support, especially the core, and the electrically conductive first layer. The second layer may also be configured to facilitate connection between the support, especially the core, such as a steel support (core), and the electrically conductive first layer. In this way, the all over conductivity can be improved.

Optionally, between the support and the electrically conductive second layer one or more further layers may be present, such as a Teflon layer. However, in embodiments especially the electrically conductive first layer is configured directly on the electrically conductive second layer; i.e. without intermediate layers between the electrically conductive second layer and the electrically conductive first layer. In a specific embodiment the electrically conductive second layer comprises copper. Other materials than copper may also be applied. In a specific embodiment, the electrically conductive second layer comprises one or more of Copper, Silver, Brass, Invar, Kovar, Steel, Stainless steel, Titanium, and Aluminium. Alternatively or additionally, the electrically conductive second layer comprises nickel. Also alloys of two or more metals may be applied (such as Kovar or Invar). Copper, and other metals, may for instance be provided by chemical vapour deposition of physical vapour deposition.

In an embodiment, the electrode has a diameter of 4 mm or less. In yet a further embodiment, the electrode has a diameter of 100 µm or less, even more especially a diameter of 60 µm or less. Especially, the diameter of the electrode is at least 20 µm. Thinner electrodes may especially be suitable for e.g monitoring brain processes in animals, whereas the more thicker electrodes may especially be suitable for clinical applications in humans. Especially, the electrically conductive second layer has a layer thickness in the range of up to about 100 µm, like up to 50 µm, such as up to 20 µm. Layer thicknesses below 10 µm, such as below 1 µm may also be possible. The electrode may have a length selected from the range of 0.1 mm - 20 mm, such as a length selected from the range of 1 mm - 10 mm. However, dimensions down to about 1 µm may also be possible, such as 1-200 µm, or even 0.1-20 µm.

As indicated above, especially an enzyme is used to measure biological molecules (and/or other molecules) by their reaction product(s) from an enzymatical reaction with the enzyme. Hence, the biosensor unit further comprises an enzyme attached to the electrode, wherein the enzyme is attached to the exclusion layer. Hence, the exclusion layer may be coated with enzymes. Therefore, the exclusion layer may be in contact with an enzyme layer.

For first generation sensors, enzymes might be linked via cross linkers like PEDGE, glutaraldehyde or glutardialdehyde with stabilizing molecules like albumine before adhesion to the electrode. For second generation sensors, enzymes might be either fixed by co-entrapment with redox mediators in different layers, or linking or electrical wiring with redox polymeres using for instance linkers like PEDGE. In third generation sensors, enzyme and mediator immobilization methods include the layer-by-layer deposition of polyelectrolytes, creating hydrogels and electropolymerization in the presence of the enzyme and the mediator to trap them at the electrode surface. In third generation sensors, electrode surfaces might be modified using nanomaterials like gold nanoparticles, carbon nanotubes and graphene nanomaterials. Deposition of enzymes, linkers, polymeres, etc. might occur through dipcoating, microprinting, electropolymerization, chemical polymerization and light induced polymerization. Eventual loading of enzymes on sensors might depends on deposition methods, but may range from 0.0001 Units/mm² to 10 Units/mm². Here, the area especially refers to the electrode area, more especially to the area of the electrode that is occupied with a (functional) enzyme. The enzymes may form a(n enzyme) layer on the exclusion layer. Hence, in an embodiment the protective layer may be permeable for bulky chemical molecules (including charged molecules), such as one or more of ascorbate, 3,4-dihydroxyphenylacetic acid (DOPAC), dopamine, and uric acid.

Molecules of interest (i.e. analytes) (in human tissue) may e.g. be selected from the group comprising one or more of cholines, alcoholes, ascorbate, aspartate, cholesterol, galactose, glucose, glutamate, glycerol, lactate, pyruvate, etc. etc. Hence, especially the enzyme comprises one or more of an Acetylcholinesterase, Choline oxidase, Alcohol oxidase, D-amino acid oxidase, L-amino acid oxidase, Ascorbate oxidase, Aspartate oxidase, Catalase, Cholesterol esterase, Cholesterol Oxidase, Galactose oxidase, Glucose oxidase, L-glutamate oxidase, GABase, Glutaminase, Glycerol kinase, Glycerol-3-phosphate oxidase, Glycerol-3-phosphate oxidase, Hexokinase, Horseradish peroxidase, Lactate oxidase, pyruvate oxidase, and Lysine oxidase. For certain applications, combinations of enzymens might be warranted (2^{nd} and 3^{rd} generation sensors, or GABA sensors). Herein, the term "attached to" may also refer to "deposited on". In a specific embodiment, the biosensor is configured to sense lactate. In yet another embodiment, the biosensor is configured to sense glutamate. In yet a further embodiment, the biosensor is configured to sense choline.

Optionally, a further layer may be available which may be applied over the enzymes (over the enzyme layer). Such protective layer may for instance be Nafion. Other materials that may be applied alternative to Nafion or in additon to Nafion may be selected from the group consisting of celluloses, such as cellulose acetate. Poly Urethane, might also be used as protective layer, also in combination with Nafion. Hence, in an embodiment the electrode further comprises a protective layer configured to enclose the enzyme (layer). The protective layer may be configured as membrane. The protective layer may be permeable for biomolecules. Hence, the protective layer may be configured to protect the enzymes or enzyme layer, but also be configured to provide access to the enzyme(s).

In general to at least one electrode as described above, also a reference electrode will be available. Such reference electrode may be an electrode arranged spatially remote from the electrode. However, in embodiments the reference electrode may also be connected to the electrode, even wired around. As will be clear to the person skilled in the art, the electrode and reference electrode will be configured in electrical insulation from each other. To this end, the electrode may further partially comprise an electrical insulating coating (such as Teflon), on which the reference electrode may be configured. Hence, in a further embodiment the biosensor further comprises a reference electrode, more especially the reference electrode comprises an Ag/AgCl electrode. Another reference elctrode that may be applied may be selected from the group consisting of a standard hydrogen electrode (SHE) (E=0.000 V) (activity of H+=1), a Normal hydrogen electrode (NHE) (E ≈ 0.000 V) (concentration H+=1), a Reversible hydrogen electrode (RHE) (E=0.000 V - 0.0591 *pH), a Saturated calomel electrode (SCE) (E=+0.241 V saturated), a Copper-copper (II) sulfate electrode (CSE) (E=+0.314 V), a Silver chloride electrode (E=+0.197 V saturated) (see above), a pH-electrode (in case of pH buffered solutions), a Palladium-hydrogen electrode, a Dynamic hydrogen electrode (DHE), and an Iridium Oxide reference electrode.

As the electrical signals may be relatively small, an amplifier may have to be used. In order to improve the signal, it may be desirable to use a preamplifier, especially a preamplifer that is included in the biosensor, such as included in the biosensor unit. Hence, in an embodiment, the biosensor further comprises a preamplifier in functional connection with the biosensor unit and functionally connected to said biosensor unit. Such that the preamplifier may be a disposable preamplifier. In an embodiment, the preamplifier can be plugged in the biosensor unit (like a plug-socket connection). Hence, in an embodiment the preamplifier is detachable connect to the biosensor unit. In yet a further aspect, the invention also provides such (disposable) preamplifer per se, wherein such preamplifer is configured to pre amplify an electrical signal measured via the biosensor unit as defined herein.The preamplifier might be of OPAMP technology, CMOS technology or other integrated technology.

The biosensor, especially the biosensor unit, may have dimension that facilitate for instance use as sensor for monitoring a baby during delivery. Hence, in an embodiment the biosensor, especially the biosensor unit, may have outer dimensions which are equal to or smaller than 20 mm (i.e. length, widht, height, diameter, etc.).

In an embodiment, the biosensor unit may include an array of electrodes. For instance, the biosensor unit may include 2-20 electrodes. In addition, the biosensor unit may include the above-mentioned reference electrode. In an embodiment the biosensor may comprise at least two electrodes, wherein the electrodes have a shortest distance of 80 µm or less, such as having a shortest distance of 50 µm or less. This may especially be of relevance for central nerveous system applications. However, the smallest distance may especially be at least 1 µm, such as at least 5 µm. In an embodimen, the biosensor may comprise at least six electrodes (especially having such inter electrode distances). However, larger distances, such as for a lactate sensor, such as for fetal applications, the distance may be larger, such as at least 0.5 mm, like in the range of 0.5-5 mm. An array of electrode may also allow embodiments wherein different enzymes may be applied.

In yet a further aspect, the invention also provides a sensing system, as further defined in the accompanying claims, comprising the biosensor as defined herein, wherein the biosensor comprises at least two electrodes, wherein the sensing system further comprises a source of electrical energy in functional connection with the at least two electrodes, and a detector configured to measure an electrical signal between at least two of the at least two electrodes. For instance, a potentiostate may be applied having the functionality of a source of electrical energy and the functionality of a detector. Here, the at least two electrodes may in an embodiment also comprise a reference electrode. Hence, the sensing system comprises at least one electrode as defined herein, and a reference electrode. In an embodiment, the sensing system is especially configured to perform amperometry with the electrode of the biosensor, especially in combination with a reference electrode. Alternatively or additionally, the sensing system is especially configured to perform one or more of voltametry and coulometry. Hence, the biosensor unit is especially suitable for one or more of amperometry, voltametry and coulometry (when used in combination with a reference electrode).

During operation, a potential difference may be applied to the electrode and the reference electrode. Especially, the potential difference is such that the electrode might be configured as positive electrode. Especially, the potential difference may be in the range of - 1V -+ 1V (relative to the reference electrode).

As indicated above, the biosensor may have different configurations. The above configurations are not limiting. The biosensor may be configured as first, second or third generation biosensor.

For instance, as known in the art the first generation glucose biosensors estimated glucose concentration in the sample based on hydrogen peroxide production by glucose oxidase (GOx) utilizing dissolved oxygen as given below:

Glucose + O₂ → gluconic acid + H₂O₂

A positive potential is applied to the Pt working electrode for a reductive detection of the oxygen consumption as:

H₂O₂ → O₂+2H+ + 2e-

Thus, the rate of oxydation of hydrogen peroxide is directly proportional to the glucose concentration. Hydrogen peroxide thus produced as a byproduct is oxidized at platinum (Pt) anode. The electrons transferred are recognized by electrode and thus the number of electrons transferred is directly proportional to the number of glucose molecules present. In the second generation biosensors one may further use a (synthetic) mediator. Examples of such mediators are e.g. Vinyl ferrocene, [Fe(CN)6]4-, Indigo Disulfonate, Methylene blue, 1,1- dimethyl ferrocene, [Ru(CN)6]4-, TCNQ, Ferrocene carboxylic acid, Ferrocene carboxaldehyde, TTF, Benzyl viologen, Hydroxy methyl ferrocene, Ferrocene, N-ethyl phenazene, and TMPD. The electrons released during an enzymatic conversion are picked by the mediator and is reduced; finally at the applied potential oxidation of mediator releases electrons that are transferred to the electrode. The role of a mediator is facilitating electron transfer. Such mediater is also immobilized on the electrode, such as embedded in a polymeric layer. In the third generation biosensor, the prosthetic group of the enzyme has direct electrochemical contact with the surface of the sensor (i.e. in embodiments on the exclusion layer on the electrically conductive first layer). Examples of those enzymes are horseradish peroxides, lactoperoxidase or microperoxidase (see also above). Third generation sensors might use nanomaterials like Gold nano particles, Carbon nano particles, Graphene nanomaterials and nanowires to facilitate enzyme immobilization and further imrpove electron transfer between the redox center of the enzyme and the electrode.

The biosensor may be applied for all kind of applications. Especially, the biosensor as described herein or the sensing system as described herein, may be used for sensing biomolecules in the tissue, like skin tissue, of a baby during giving birth. Examples of such biomolecules are lactate and a relevant enzyme may be lactate oxidase. Further, the biosensor as described herein or the sensing system as described herein, may be used for sensing neurotransmitters and/or metabolites (herein also indicated as endogenous analyte (and metabolite theroef, although such metabolite may thereby also be considered to be an analyte), like glutamate, aspartate, D-serine, γ-aminobutyric acid (GABA), glycine, monoamines and other biogenic amines like dopamine (DA), norepinephrine (noradrenaline; NE, NA), epinephrine (adrenaline), histamine, serotonin (SE, 5-HT), peptides like somatostatin, substance P, opioid peptides, and molecules like acetylcholine (ACh), adenosine, anandamide, nitric oxide, lactate, pyruvate, glucose, choline, etc., in the tissue of an animal, such as a mammal. Hence, the invention provides the use of an electrochemical active coating on a hard rigid material for application of biosensors in vivo. The invention also provides the use of a gold coating on stainless steel carrier material for measuring lactate in fetal scalp during labor. Especially, the invention also provides the use of a platinum coating on a tungsten needle for measuring neurotransmitters in tissues of an animal. Hence, this invention provides amongst others the use of platinum coated tungsten sensors in conjunction with disposable pre amplifiers for monitoring (brain) neurotransmitter levels.

Below, some embodiments are described in more detail:

| | **First type of application** | | **Second type of application** | |
|---|---|---|---|---|
| | **Application 1 Embodiment 1** | **Application 1 Embodiment 2** | **Application 2 Embodiment 1** | **Application 2 Embodiment 2** |
| Eample of application | (Rat) Brain | (Rat) Brain | (Fetal) Scalp | (Fetal) Scalp |
| Analyte(s) to be sensed | Glutamate | Choline | Lactate | Lactate |
| Core material | Tungsten | Tungsten | Stainless steel | Stainless steel |
| Second electrically conductive layer (if any) material | Copper | Copper | - | Copper |
| Thickness layer Second electrically conductive layer (if any) | 1 µm | 1 µm | | 1 µm |
| First electrically conductive layer material | Pt | Pt | Gold | Gold |
| Layer thickness first electrically conductive layer | 5 µm | 5 µm | 5 µm | 5 µm |
| Exclusion layer (such | Nafion/PPD | Nafion/PPD | Nafion | Nafion |
| Eample of application | (Rat) Brain | (Rat) Brain | (Fetal) Scalp | (Fetal) Scalp |
| as nafion) material | | | | |
| Thickness exclusion layer | 1 µm | µm | µm | µm |
| Enzyme | Glutamate oxidase | Choline oxidase | Lactate oxidase | Lactate oxidase |
| Number of electrodes, including reference electrode | 2 | 2 | 2 | 2 |
| Type of counter electrode | Ag/AgCl | Ag/AgCl | Ag/AgCl | Ag/AgCl |

Hence, the invention provides amongst others the use of an electrochemical active coating on a hard rigid material for application of biosensors in vivo. The invention also provides such use, especially of a gold coating on stainless steel carrier material suitable for measuring lactate in fetal scalp during labor, wherein optionally copper is applied as electrically conductive second layer. Alternatively, the invention also provides such use, especially of a platinum coating on stainless steel carrier material suitable for measuring lactate in fetal scalp during labor, wherein optionally copper is applied as electrically conductive second layer. Alternatively, the invention also provides such use, especially of a platinum coating on a tungsten needle suitable for measuring neurotransmitters in brains of an animal, wherein optionally copper is applied as electrically conductive second layer. The application further discloses, in an example not forming part of the claimed invention, a method for measuring a biomolecule in an animal, the method comprising using the biosensor or the sensing system as defined herein, arranging the electrode in animal tissue, applying one or more of amperometry, voltametry and coulometry, especially applying a voltage difference between the electrode and a reference electrode, and monitoring the current as function of time.

In yet a further aspect, the invention also provides a method for making an electrode for a biosensor for sensing an endogenous analyte as defined herein, by constructing a sensor surface on a biocompatible material meant for invasive procedures in humans and/or animals, wherein the sensor surface at least comprises an electrically conductive first layer and an exclusion layer, wherein the electrically conductive first layer is
configured between the biocompatible material and the exclusion layer, as further defined in the accompanying claims. Hence, in a further aspect the invention provides a method for making a biosensor, the method comprising: (i) providing a support, especially a (bio compatible) hard material, in an embodiment an electrically conductive support, (ii) optionally providing an insulating layer (on the support), (iii) optionally providing an electrically conductive second layer (on the support or insulating layer, respectively), (iv) providing the electrically conductive first layer (on the support, the insulating layer, or the electrically conductive second layer, respectively), (v) providing an exclusion layer on the electrically conductive first layer, (vi) optionally providing enzymes on the electrically conductive first layer, and (vii) optionally providing a protective layer (configured to substantially enclose the enzymes (or enzyme layer)). Hence, the biosensor, or more especially the biosensor unit or electrode may comprise a multi-layer system on a support, with especailly at least an exclusion layer on the electrically conductive first layer. The electrode may thus comprise a plurality of functional layers, with especailly at least an exclusion layer on the electrically conductive first layer, even more with especailly at least an exclusion layer on the electrically conductive first layer, and an enzyme layer on the exclusion layer. As will be clear to a person skilled in the art, adjacent layers (having different functionality) also have different compositions. For instance, assuming a copper support, there may be no need for an electrically conductive second layer (of copper), whereas a titanium support may comprise a copper electrically conductive second layer.

The invention especially provides a peroxide-responsive biosensor comprising: a tungsten core; and a platinum coating applied to the tungsten core. Further, especially such biosensor may have a thickness less than about 50 micrometers. The invention further provides a system for detecting a biomolecule, especially a neurotransmitter, in animal tissue, such as the brains comprising: a peroxide-responsive biosensor including: a tungsten core; and a platinum coating applied to the tungsten core; and disposable preamplifier coupled to the biosensor. Yet, the invention also provides a biosensor comprising: a rigid material forming a core; and a platinum coating applied to the core. Further, such biosensor may have a thickness less than about 50 micrometers. Also, the invention provides a system for detecting a biomolecule, especially a neurotransmitter, in animal tissue, such as the brains comprising: a biosensor including: a rigid material forming a core; and a platinum coating applied to the core; and disposable preamplifier coupled to the biosensor. Further, the invention provides the application of hard rigid materials like tungsten that can be coated by platinum in order create peroxide responsive biosensors. These peroxide responsive sensors are used to create enzyme based biosensors for in vivo applications. In yet a further aspect, the invention describes the application of other rigid materials like, but not limited to tungsten, Ag₂O, stainless steel, etc., alone or coated by Pt or other electrochemically active coatings. In yet a further aspect, the invention also provides the use of disposable preamplifiers in conjunction with small rigid material based biosensors in order enable sufficient signal-to-noise ratio for biosensor measurements, routinely using biosensors< 50 micrometer. The invention also provides the combination of using disposable preamplifiers with Pt coated tungsten sensors as a crucial combination to perform biosensors experiments in vivo with biosensors with a spatial resolution of less than 50 micrometer in diameter. In order to facilitate the use of these smaller platinum-coated tungsten sensors, a disposable preamplifier (see examples) is used, and enables pre-amplification of sensor signals without the risk of losing signal due to imperfect connections between sensors and reusable preamplifiers. The use of these disposable preamplifiers significantly reduced the presence of noise in vivo. Hence, the invention also provides the use of platinum-coated tungsten sensor in conjunction with disposable preamplifier for monitoring brain neurotransmitter level.

This application claims the priority of US provisional application 61702,098.

While embodiments and applications have been shown and described, it would be apparent to those skilled in the art having the benefit of this disclosure that many more modifications than mentioned above are possible without departing from the inventive concepts claimed herein. Those of ordinary skill in the art will realize that the following description is illustrative only and is not intended to be in any way limiting. Other embodiments, within the scope of the claims, will readily suggest themselves to such skilled persons having the benefit of this disclosure. Reference will now be made in detail to implementations of the example embodiments as illustrated in the accompanying drawings. The same reference indicators will be used to the extent possible throughout the drawings and the following description to refer to the same or like items. In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another. Moreover, it will be appreciated that such a development effort might be complex and time-consuming, but would nevertheless be a routine undertaking of engineering for those of ordinary skill in the art having the benefit of this disclosure. The term "substantially" herein, such as in "substantially all light" or in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species". Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The devices herein are amongst others described during operation.

In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage. The invention further applies to a device comprising one or more of the characterizing features described in the description and/or shown in the attached drawings.

### BRIEF DESCRIPTION OF THE DRAWINGS

Embodiments of the invention will now be described, by way of example only, with reference to the accompanying schematic drawings in which corresponding reference symbols indicate corresponding parts, and in which:

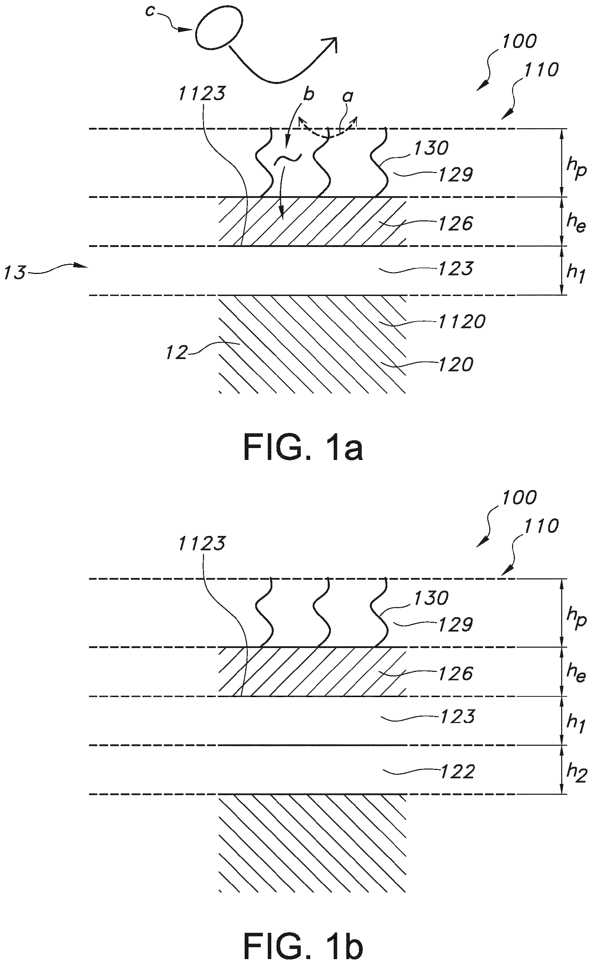

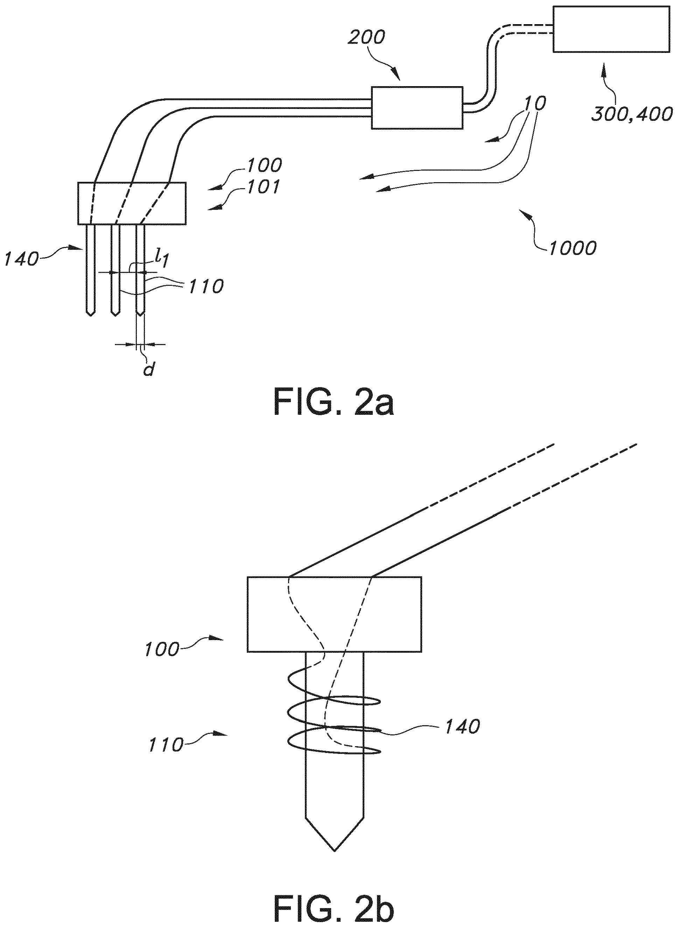

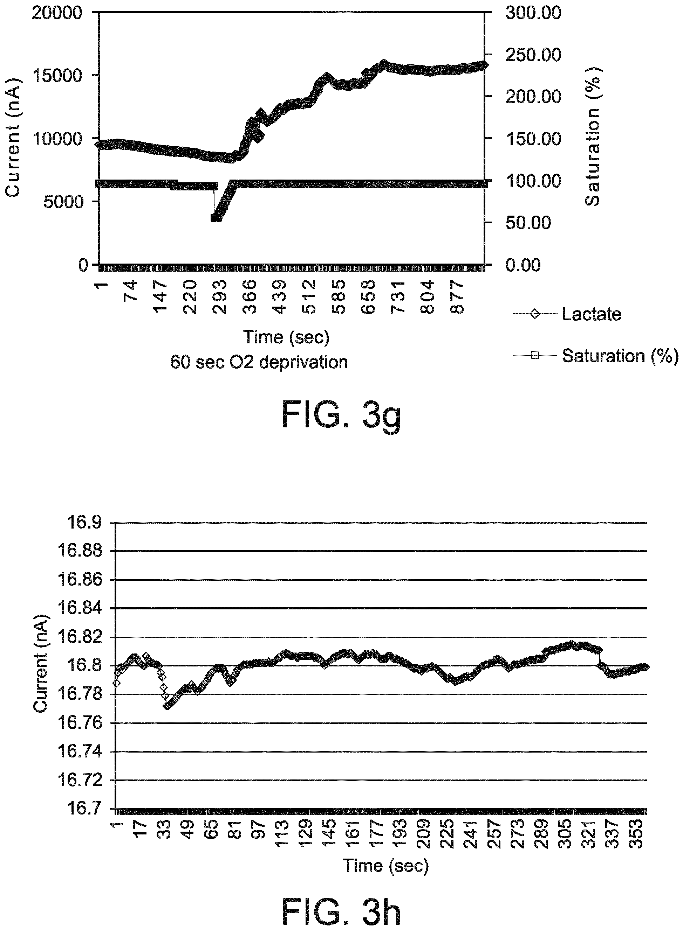

Figs. 1a-1f schematically depict some aspects of the biosensor unit, especially the electrode; Figs. 2a-2e schematically depict some embodiments of the biosensor. The drawings are not necessarily on scale; Fig. 3a shows a typical spontaneous change of noise when the activity state of an animal changes; Figs. 3b-3e show some further aspects; Figure 3f shows the response of the lactate sensor to cumulative concentrations of lactate in vitro; Figure 3g shows the response of lactate to transient deprivation of oxygen as measured subcutaneously in a rat; and Figure 3h shows an example of Glutamate signal of 50 µm Pt coated tungsten sensor with disposable preamplifier in brain of freely moving rat with changing activity states.

### DETAILED DESCRIPTION OF THE EMBODIMENTS

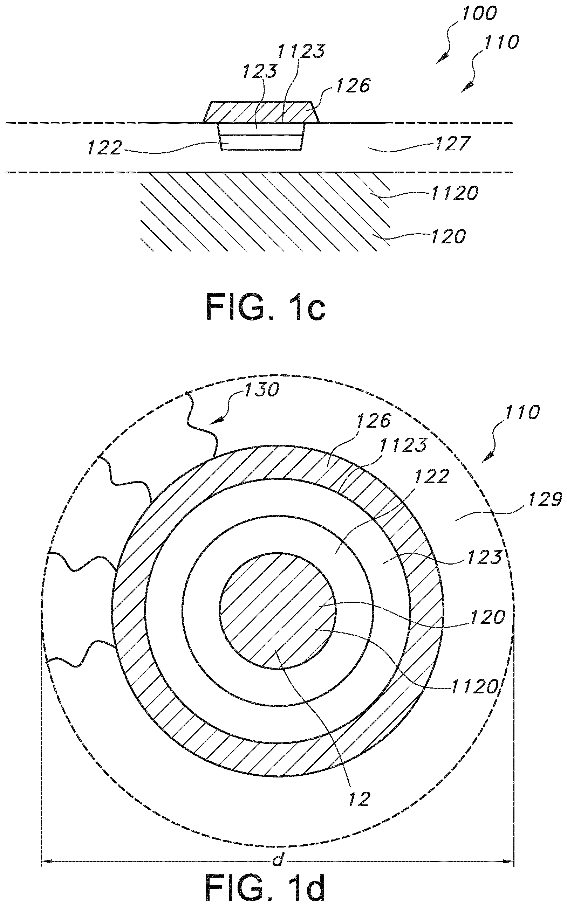

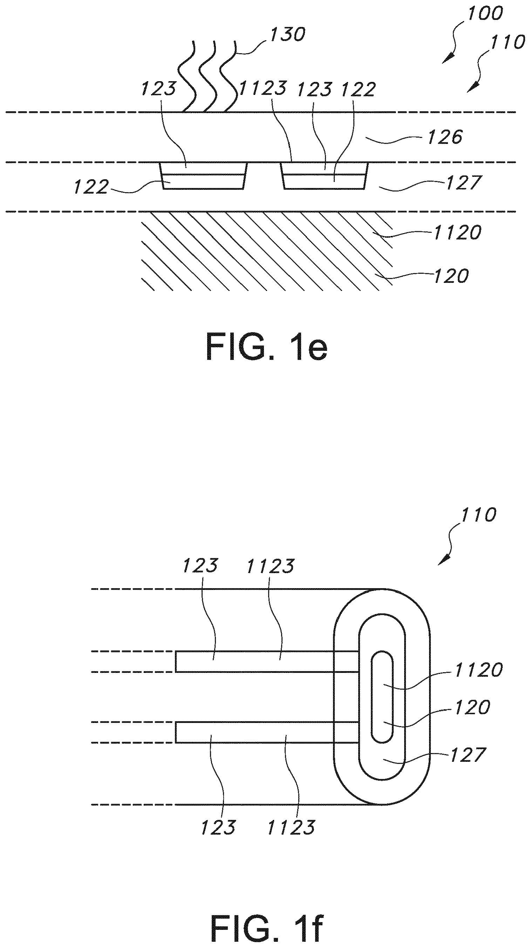

Figs. 1a-1f schematically depict embodiments and variants of a biosensor 10 comprising a biosensor unit 100 with an electrode 110. The electrode 110 can be rod-shaped (see fig. 1d). The electrode 110 further comprises a support 1120, such as a core 120 (see fig. 1d), such as tungsten (W), with an electrically conductive first layer 123, such as gold (Au) or silver (Ag), etc., and an exclusion layer 126, such as Nafion. The electrically conductive first layer 123 is configured between the support 1120, such as the core 120 and the exclusion layer 126. The thickness of the electrically conductive first layer 123 is indicated with reference h1; the thickness of the exclusion layer 126 is indicated with reference he. Further, enzymes 130 are attached to the electrode 100, especially to the exclusion layer 126. These enzymes may convert bio molecules into species that can penetrate the exclusion layer and be detected by the electrochemically active layer, i.e. the electrically conductive first layer 123. Reference 1123 indicates the surface of electrically conductive first layer 123. Referring to fig. 1a, molecule may e.g. indicate a bio molecule that is converted into other species, like e.g. lactate and H₂O₂ (the latter is indicated with ref. b), respectively, when a specific lactate converting enzyme is applied. In such instance, another bio molecule c, such as ascorbate, will not contribute to the electrochemistry and will be kept away from the electrically conductive first layer 123 due to the exclusion layer 126. Whereas lactate may not penetrate through the exclusion layer 126, H₂O₂ may do and reach the surface 1123 of the electrically conductive first layer 123. Here, H₂O₂ may be converted and generate an electrical signal. Reference 13 indicates a sensor surface (i.e. the stack of materials that provide relevant sensor functionality) and reference 12 indicates a biocompatible (especially hard rigid) material, such as tungsten (see above). Fig. 1a also schematically depicts an embodiment wherein the electrode 110 further comprises an optional protective layer 129 configured to enclose the enzyme. This layer may especially be a membrane like layer, comprising cellulose or Nafion. This layer may protect the enzymes, and enclose them, but may allow biomolecules such as lactate enter. The layer height is indicated with reference hp; the height may be in the range of 0.1-50 µm. This protective layer may be permeable for biomolecules such as ascorbate, uric acid, etc. Fig. 1b schematically depicts an embodiment with a further layer, an electrically conductive second layer 122, such as copper (Cu), between the support 1120, and the electrically conductive first layer 123, which may add to electrical conductivity to an analysis unit or detector (see also below). Fig. 1c schematically depicts an embodiment wherein one or more of the conductive layer(s) and the exclusion layer are at least partially embedded in a third layer 127 of biocompatible material, such as Teflon. In such instance, there may be no direct contact between the support 1120 and at least parts, or even the entire, length of the conductive layer(s). Fig. 1d shows a substantially round electrode, here in cross-sectional view. Reference d indicates the diameter. Especially, the third layer 123 comprises an insulating material. Fig. 1e schematically an embodiment wherein the electrode comprises a plurality of active sites. Here, two electrically conductive first layer 123 are available, with each an electrically conductive second layer 122. Both are covered by the exclusion layer 126. However, enzymes 130 are only applied to one of the active sites (left site). The other site may e.g. be used as reference. The optional protective membrane is not depicted (for clarity reasons). In this embodiment, the electrode in fact comprises two (or more) electrodes in one unit. Hence, a single electrode rod may comprise in embodiments two or more electrodes. In such instance, the electrode 110 may also be indicated as electrode unit with two or more conductive surfaces 1123, which may be electrically insulated from each other. Fig. 1f schematically depicts a similar embodiment, but now in a rod-shaped version. Such embodiment may also be indicated as multi-array electrode. Such electrode may comprise a plurality of active sites where analytes (or their metabolites) may be measured. An array of may also allow embodiments wherein different enzymes may be applied.

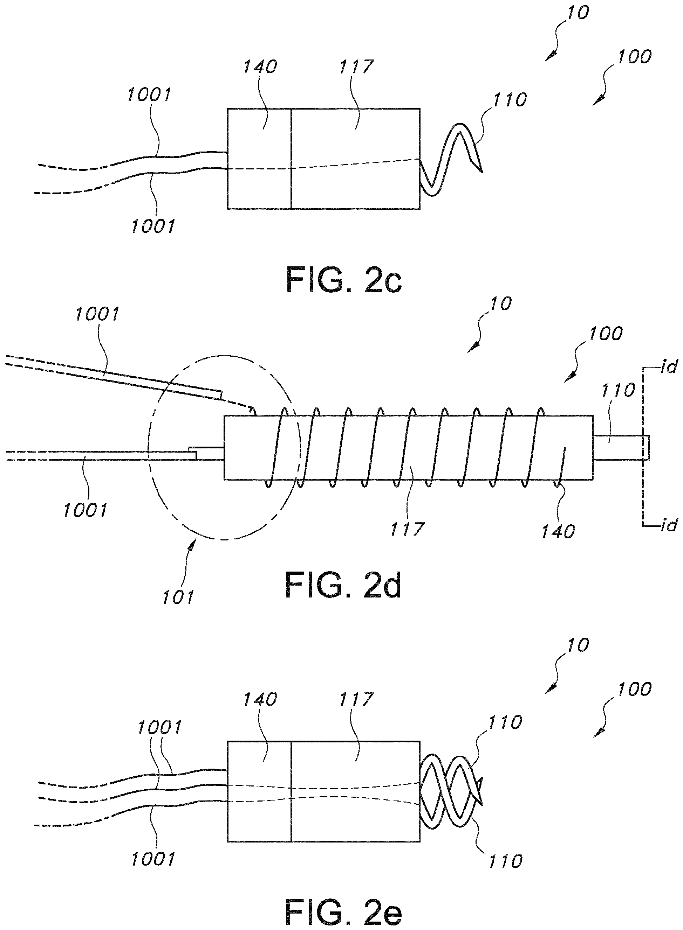

Fig. 2a schematically depicts a sensing system 1000, with an optional preamplifier 200, which may be slightly remote from the electrodes, or which may be connected to a board or support 100 configured to support the electrode(s) 110. The biosensor unit 110 is electrically connected to a source of electrical energy 300, such as a potentiostate including a detector 400, which may be a single unit, such as a (Pinnacle) potentiostate . Here, by way of example two electrodes 110 are indicated, with a mutual distance 11. Further a reference electrode 140 is drawn. All electrodes are electrically connected to the source of electrical energy 300 and the detector 400. Fig. 2b schematically depicts an embodiment of the biosensor unit 100 with a counter (or reference) electrode, such as an Ag/AgCl electrode, which is wired around the electrode 110. Figure 2c schematically depicts an embodiment of the biosensor unit 100with a Ag/AgCl reference electrode 140 is present and a coiled electrode 110 can be used for insertion in the skin. Fig. 2d schematically depicts an alternative embodiment. Note that the reference electrode 140 is coiled around the electrode 110. The biosensor unit further comprise an insulation layer 117, covering at least part of the electrode 110. Around this insulating layer 117, the reference electrode 140 may be coiled. An embodiment of a possible cross-section of the electrode 110 can be found in fig. 1d). Fig. 2e schematically depicts an alternative embodiment with two electrodes 110. Both may be covered with enzymes, though optionally one may not be covered with enzymes for reference purpose. Configuration like 2c and 2e may be of special interest when arranging such electrode in the body, such as in a skull. Hence, such bio sensor may especially be applied as fetal sensor. For instance, the embodiment of fig. 2e may be applied as double wire fetal scalp electrode. Fig. 3a shows a typical spontaneous change of noise when the activity state of an animal changes. Signals 1 and 2 present glutamate signals of a commercial multi electrode sensor and 3 and 4 represent the back ground sensors without glutamate oxidase. Figs. 3b-3e show some further aspects of the preamplifier. Figure 3f shows the response of the lactate sensor to cumulative concentrations of lactate in vitro (see also below). Figure 3g shows the response of lactate to transient deprivation of oxygen as measured subcutaneously in a rat. Fig. 3h shows an example of Glutamate signal of 50 µm Pt coated tungsten sensor with disposable preamplifier in brain of freely moving rat.

### EXAMPLES

### Example 1: Sensitivity of Pt coated tungsten sensors to peroxide

In one experiment the H202 sensitivity of platinum coated tungsten wires was tested, in order to study whether it would be possible to use them as biosensors. Table 1 shows the responsiveness of 50-micrometer thick (diameter) platinum-coated sensors to H202, in comparison to conventional 200 micrometer thick platinum wire sensors. The production procedure for the novel platinum-coated tungsten sensors is described in appendix A. These 50-micrometer thick (diameter) platinum-coated sensors have good responsiveness to H202 as Table 1 shows. In addition, platinum-coated tungsten is very rigid, so remains intact during insertion in the tissue, such as brain at diameters below 50 micrometers. Platinum wire electrodes, by comparison, typically do not survive these insertions, especially not after insertion in freely moving animals.

**Table 1: Sensitivity of Pt coated tungsten sensors to H2O2**

| | Pt200-GluS1 | Pt200-GluS2 | PtTungsten50 | PtTungsten50 |
|---|---|---|---|---|
| coating | Nafion-GluOx-PPD | Nafion-GluOx-PPD | Nafion | Nafion |
| | | | | |
| slope (nA/µM) | 0.946182 | 1.248459 | 0.766244 | 0.674083 |
| R-squared | 0.999439 | 0.999998 | 0.999364 | 0.999861 |
| LOD (µM) | 0.031685 | 0.032701 | 0.418896 | 0.258404 |
| LOD (µM; normalized) | 0.126738 | 0.130806 | 0.418896 | 0.258404 |

### Example 2: Assembly of a sensor

| | Order form Assembly of standard coiled biosensor |
|---|---|
| | Step 1: Part assembling sensor |
| Day 1 | Assembling |
| 1.1 | Cutting: TSP075150; L=12mm |
| | Cut Pt Plated tungsten wire (P069) (OD = 0.05 mm; L=16mm) in L = 15 m |
| | Fill the silica tube with wire. |
| 1.2 | Prepare two component epoxy glue in 1 ml syringe. Fill the silica tubing containing Pt wire with epoxy glue. Make sure that there are no air bubbles inside the silica tubing. |

| Day 2 | Put the glued shaft for a minimum of 24 hours in the stove at 70°C |
|---|---|
| 1.3 | Take out the glued silica tubes from the oven. Check if the PT wire is fixed well inside by pulling the wire. |
| | Cut one end of the Pt wire to 1 mm and 2 mm precisely |
| | Clean the sensors by immersing in: |
| | 1. 5 minutes in acetone |
| | 2. 10 minutes in UP water |
| | 3. Dry the sensors at 40C for 15 minutes |
| 1.4 | Prepare Ag wire (OD = 0.05 mm) in a roll and place it on a stick to hold the round. Under the microscope place a small drop of pattex glue on silica tube opposite end of the sensor tip. Place Ag wire very gently on the glue and let it dry for 3 minutes. Coiled L = 9mm. |
| | Start to make Ag coil on top of silica tubing until 1 mm from the sensor end and glue the Ag on the silica tubing. Let it dry for 3 minutes. Cut the remaining Ag wire from the glue. |
| 1.5 | Prepare 2 insulated copper wire with different color: red is for the working electrode and gold for the reference electrode. |
| | Red wire L = 20m, Gold wire L = 20 mm |
| | Unleash 1 m of each end of the copper wire. Under the microscoop melt a small piece of tin on copper surface and connect to the corresponding wire. |
| | Check the R after soldiering process (should not be more than 10 ohm) |
| | Place the Pt-silica-Ag coil and copper wires in the microdialysis body; expose Pt-silica-Ag coil 8 mm from microdialysis body. |
| | Use UV glue to fill MD body and cure it under UV light. |

| Day 3 | Preparing Ag/AgCl electrode |
|---|---|
| 1.6 | Coat Ag with Ag Cl by the following procedure: |
| | a. Prepare a bottle containing solution HCL 0.1M NaCl saturated and battery source with 9V. |
| | b. Connect the lead (fro Ag wire) to (+) and stainless steel to (-) |
| | c. Place the sensor in solution together with stainless steel |
| | d. Chloride coating process on Ag surface takes 10 minutes. |
| 1.7 | Clean the sensors thoroughly with UP. |
| 1.8 | Place a semi-transparant tube to cover Ag coil and also sensor to avoid physical damage. |

| Assembly | |
|---|---|
| Biosensor | |
| Day 4 | Step 2: Nafion coating |
| 2.1 | Heat up the oven 180C +/- 45 minutes before nafion coating. |
| 2.2 | Prepare nafion solution by placing +/- 1.50 ml in 1.5 ml vial. |
| 2.3 | Coating process: immerse Pt wire (and also Ag/Ag coil for Pt-Ag coil) in nafion solution for 10 seconds and followed by drying in the air for 20 seconds. Repeat this procedure for 5 minutes. |
| 2.4 | By placing in metal-net plate, bake the sensors at 180°C for 4 minutes. |
| 2.5 | Take out the sensors from the oven and keep in room temperature for 5-10 minutes. |
| 2.6 | Place the sensors in box. Keep 3 h at least room temperature for curing process prior to enzyme coating. |
| | Step 3: Enzyme coating |
| 3.1 | Find bovine serum albumine (BSA) and glutaraldehyde in fridge. Set BSA (4 °C, KK1) and glutaraldehyde (-18 °C, V1) at room temperature |
| 3.2 | Weight 5 mg of BSA in vial 1.5 mL. Add in gently 333 µL UP water. Turn up-down gently the vial until BSA is completely dissolved. |
| 3.3 | After BSA is dissolved, add 5mL 25% glutaraldehyde. Final concentration: BSA 10% and 0.125% glutaraldehyde. Mix solution by turning up-down the solution 5 times. Leave the solution reacting for 5 minutes. |
| | During this time unfreeze: |
| | Glutamate sensor: one vial containing 1.0 µL Glu-Ox 1 U/µL |
| | Background sensor: one vial containing 1.0 µL inactivated enzyme Glu-Ox 1 U/µL |
| 3.4 | Glutamate sensor: When mixing is ready, add 4 µL solution (BSA 10% + 0.125% glutaraldehyde) mixture to 1 µL Glu-Ox 1 U/µL. Mix gently with pipette. |
| | Background sensor: When mixing is ready, add 4 µL solution (BSA 10% + 0.125% glutaraldehyde) mixture to 1 µL inactivated enzyme Glu-Ox 1 U/µL. Mix gently with pipette. |
| 3.5 | Prepare Hamilton syringe for Glu-Ox solution and inactivated enzyme Glu-Ox solution. Place the sensors on black foam. |
| 3.6 | Under the microscope, coating the sensors (Pt wire) with a drop obtained by Hamilton syringe. Pt 25 and Pt 200 should be coated 25 and 200 times respectively. Pt 50 should be coated 50 times? |
| 3.7 | Leave the sensors at room temperature to cure the enzyme coating for at least 72 hours prior to calibration. |
| 3.8 | After coating process, clean the syringe with ethanol 95% (15 times), followed by UP water (15 times). |
| | Step 4: m-PD electropolymerization |
| 4.1 | Turn on the computer and Pinnacle machine. Turn on the water-heater to maintain the temperature at 37 °C. |
| 4.2 | Set up the Pinnacle machine for calibration as follow : |
| | 1. Click Bias (in setting menu) and type 0.7 or 0.5 for all channels. It means we apply 700 mV or 500 mV for working potential. |
| | 2. Click AD Auto zero (in setting menu). When we click AD Auto zero, all lines (current) should be in zero (calibrating process). |
| 4.3 | Preparing 5 mM m-PD (ortho-phenylene diamine) onomer of PPD in PBS solution: |
| | 1. Deoxygen 50 ml PBS 50 mM using N2 for 20 minutes |
| | 2. During deoxygen process, weight o-PD 36.2 mg in covered (with alumunium foil) volumetric flask, put magnetic stirrer inside and pour gently 40 ml PBS 50 mM in. |
| | 3. Stir the solution in a slow rate until m-PD is totally dissolved (2-5 min). m-PD could be naturally oxidized after 3 hours (turns to brown), therefore-PD should be freshly prepared before use. |
| 4.4 | Put the sensors in m-PD solution together with Ag/AgCl wire as reference electrode and Pt wire as auxiliary electrode |
| | Connect the background and the sensors to red-clips and the reference to black-clips. |
| 4.5 | 1. Start a new file on computer for calibration |
| | 2. Open CH instrument application |
| | 3. On tab menu, click File → Log → start → type file's name (for example : '20090427_ GluS-BG_1-2_3-4_precalibration') |
| | 4. Place the sensors in o-PD solution. PPD coating process takes 20 minutes. A nice smooth line with gradually decreased should be observed. |
| 4.6 | When the process is finished, take out the sensors and wash with UP water. Dry the sensors at room temperature. |
| | Step 5: Calibration procedures |
| 5.1 | Turn on the computer and Pinnacle machine. Turn on the water-heater to maintain the temperature at 37 °C. |
| 5.2 | Set up the Pinnacle machine for calibration as follow : |
| | 3. Click Bias (in setting menu) and type 0.7 or 0.5 for all channels. It means we apply 700 mV or 500 mV for working potential. |
| | 4. Click AD Auto zero (in setting menu). When we click AD Auto zero, all lines (current) should be in zero (calibrating process). |
| 5.3 | Add 50 ml PBS 50 mM in a beaker of 50 ml. Put magnetic stirrer on it. Place the beaker inside the water bath and above stirrer machine. |
| 5.4 | Connect the background and the sensors to red-clips and the reference to the black-clips |
| 5.5 | 1. Start a new file on computer for calibration |
| | 2. Open CH or PAL instrument application |
| | 3. On tab menu, click File → Log → start → type file's name (for example : '20090427_ GluS-BG_1-2_3-4_precalibration') |
| | 4. Place the sensors in PBS 50 mM solution. Leave the sensor to stabilize at least 15 minutes before calibration. |
| 5.6 | After the sensors are stable, mark 'baseline' by click File → Log → Comment, then add series compounds each 50 µL, in following order (final concentration) : |
| | 1. Dopamine (DA) 2 mM in 0.1mM HCLO4 (final concentration 2 µM) |
| | 2. Dihydroxyphenylacetic acid (DOPAC) 20 µM (final concentration 20 µM). |
| | 3. Uric acid (UA) 50 mM. |
| | 4. Ascorbic acid 250 mM. |
| | 5. Analytes (Glutamate, GABA, Choline, Acetylcholine) 2.5 (2x), 5, 10, 20, 40 mM (final concentration 2.5, 5, 10, 20, 40, 80 µM) |
| | 6. Hydrogen peroxide 20 mM. |
| | 7. Cys 50 mM. |
| | After adding the compound, leave the current about 1-2 minutes to stabilizing and mark the compound on a stable level. |
| 5.7 | 1. When finish the calibration, click File → Log → Stop. |
| | 2. To export data, open the current file and click File → Export → To a spreadsheet → type your file's name |
| | 3. Store the sensors in PBS solution before next calibration or assembly. |

### Example 3: Fetal Scalp electrode,

### Electrodes and nafion layer

Gold plated fetal scalp electrodes for CTG were used for the experiments. Electrodes were cleaned by immersing for 10 minutes in acetone followed by 10 minutes in ultrapure water. The sensors were dried at room temperature for 1 hour. The sensors were coated by nafion for 5 minutes by repeated immersing in 5% nafion (sigma), followed by 20 sec of drying on air. The nafion was baked for 2 times 3 min at 170 degrees and left for at least 2.5 hrs. before further use.

### Lactate oxidase coating

Lactate oxidase was dissolved in ultrapure water to a concentration of 1 unit per microliter (stock solution). Glutardialdehyde was used as linker together with bovine serum albumin (BSA) as an additive to stabilize enzyme coating. 6.25 mg BSA was dissolved in 0.5 ml ultrapure water. 1.6 microliter of 50% Glutardialdehyde was added and the solution was gently mixed. The solution was left for 5 min. 137 microliter of the BSA-linker solution was added to 37 microliter of lactate oxidase stock solution and gently mixed. The solution transferred to the lid of an Eppendorf cup for immersion of the fetal scalp electrodes. Fetal scalp electrodes were dipped with 10 sec pauses for 10 times, after which they were left to dry for 5 minutes. This procedure was repeated for 5 times using 2 electrodes. Electrodes were left to dry at room temperature for at least an hour before use.

### Reference electrode,

The silver / silver chloride electrode was produced from a 5 cm 250 micrometer silver wire from which the Teflon layer was removed for about 1 cm at the distal end. The silver wire was connected to a 9V battery together with a stainless steel wire. Both wires were placed in a 1 M HCl solution that was saturated with NaCl. After 10 min the silver chloride coated with completed.

### In vitro experiment

A glass beaker was filled with 50 ml 50 mM PBS and stirred. The beaker glass was kept at 37 degrees Celsius by a water bath. The sensor and reference electrode was connected to a Pinnacle potentiostate. The potentiostate was controlled by a PC with PAL software. One sensor was tested at a time. A voltage of 600 mV was applied to the sensor vs. Ag/AgCl. After stabilization of baseline for 30 min, lactate (13.3 M) was added to calibrate the sensor. Figure 3f shows the response of the lactate sensor to cumulative concentrations of lactate in vitro

### In vivo experiment

Animals (Male wistar 280-350g) were anaesthetized using 2.5 isoflurane and oxygen 600 ml/min. The belly of the animals was shaven and fetal electrodes were inserted in subcutaneously. Between the insertion sides, a small incision was made and a cavity was created for insertion of the reference electrode. After insertion of the reference electrode, it was fixed to the skin with a suture. An oxygenation monitor was attached to the tail of the animal to monitor oxygenation. Nitrogen could be applied to the animal via the inhalation setup. Lactate levels could be measured subcutaneously in anaesthetized rats. Upon reduction of oxygenation by administration of nitrogen, an increase of lactate levels was observed. A clear elevation of sensor signal upon administration of nitrogen was observed (see fig. 3g).

## Claims

1. A biosensor (10) comprising a biosensor unit (100) with an electrode (110), wherein the electrode (110) is rod-shaped, wherein the electrode (110) further comprises a support (120) with an electrically conductive first layer (123) and an exclusion layer (126), wherein the electrically conductive first layer (123) is configured between the support (120) and the exclusion layer (126), wherein the electrode (110) comprises a needle shape with a needle shape tip, wherein the support (120) comprises a material selected from the group consisting of stainless steel, a carbide, titanium, vanadium, tantalum, and tungsten metal, wherein the electrically conductive first layer (123) comprises one or more of gold, silver, silver oxide, platinum, and carbon, and wherein the biosensor unit (100) further comprises an enzyme (130) attached to the electrode (110).

2. The biosensor (10) according to any one of the preceding claims, wherein the support (120) comprises a material having compression strength of at least 20,000 kPa.

3. The biosensor (10) according to any one of the preceding claims, wherein the support (120) comprises one or more biocompatible materials.

4. The biosensor (10) according to any one of the preceding claims, wherein the electrically conductive first layer (123) has a layer thickness in the range of 3 nm - 100 µm and wherein the exclusion layer (126) has a layer thickness in the range of 1 nm-50 µm.

5. The biosensor (10) according to any one of the preceding claims, wherein the exclusion layer (126) is permeable for one or more of H₂O₂, O₂, and NO, or can facilitate electron transfer (ET) and wherein the exclusion layer (126) is impermeable to one or more of ascorbate, 3,4-dihydroxyphenylacetic acid (DOPAC), dopamine, and uric acid.

6. The biosensor (10) according to any one of the preceding claims, wherein the exclusion layer (126) comprises one or more of sulfonated tetrafluoroethylene based fluoropolymer-copolymer, N,N'-Di-[(1-naphthyl)-N,N'-diphenyl]-1,1'-biphenyl)-4,4'-diamine (NPD), and p-Phenylenediamine (PPD).

7. The biosensor (10) according to any one of the preceding claims, further comprising an electrically conductive second layer (122) configured between the support (120) and the electrically conductive first layer (123).

8. The biosensor (10) according to any one of the preceding claims, wherein the electrode (110) has a diameter of 4 mm or less.

9. The biosensor (10) according to any one of the preceding claims, wherein the electrode (110) has a length selected from the range of 0.1 mm - 15 mm or wherein the electrode (110) has a length selected from the range of 0.1-200 µm.

10. The biosensor (10) according to any one of the preceding claims, wherein enzyme comprises one or more of an Acetylcholinesterase, Choline oxidase, Alcohol oxidase, D-amino acid oxidase, L-amino acid oxidase, Ascorbate oxidase, Aspartate oxidase, Catalase, Cholesterol esterase, Cholesterol Oxidase, Galactose oxidase, Glucose oxidase, L-glutamate oxidase, GABase, Glutaminase, Glycerol kinase, Glycerol-3-phosphate oxidase, Glycerol-3-phosphate oxidase, Hexokinase, Horseradish peroxidase, Lactate oxidase, Pyruvate oxidase, and Lysine oxidase.

11. The biosensor (10) according to any one of the preceding claims, wherein biosensor (10) is configured as first, second or third generation biosensor.

12. The biosensor (10) according to any one of the preceding claims, further comprising a protective layer (129) configured to enclose the enzyme.

13. The biosensor (10) according to any one of the preceding claims, further comprising a reference electrode (140), wherein the reference electrode comprises an Ag/AgCl electrode.

14. The biosensor (10) according to any one of the preceding claims, comprising a preamplifier (200) in functional connection with the biosensor unit (100) and functionally connected to said biosensor unit (100).

15. The biosensor (10) according to any one of the preceding claims, wherein the exclusion layer (126) is permeable for H₂O₂, wherein the exclusion layer (126) comprises Nafion, wherein the support (120) comprises a metal or metal alloy having a shear strength of at least 40 GPa, wherein the electrode (110) has a diameter of 100 µm or less, the biosensor (10) further comprising an electrically conductive second layer (122) configured between the support (120) and the electrically conductive first layer (123), wherein the electrically conductive second layer (122) comprises copper.

16. The biosensor (10) according to any one of the preceding claims, having outer dimensions which are equal to or smaller than 20 mm.

17. The biosensor (10) according to any one of the preceding claims, comprising at least two electrodes, wherein the electrodes have a shortest distance of 80 µm or less, or wherein the electrodes have a shortest distance of at least 0.5 mm.

18. The biosensor (10) according to any one of the preceding claims, comprising at least six electrodes (110).

19. A sensing system (1000) comprising the biosensor (10) according to any one of the preceding claims, wherein the biosensor (10) comprises at least two electrodes, wherein the sensing system (1000) further comprises a source (300) of electrical energy in functional connection with the at least two electrodes, and a detector (400) configured to measure an electrical signal between at least two of the at least two electrodes.

20. A method for making an electrode (110) for a biosensor (10) for sensing an endogenous analyte as defined in any one of claims 1-18, by constructing a sensor surface on a biocompatible material (12) meant for invasive procedures in humans and/or animals, wherein the sensor surface (13) at least comprises an electrically conductive first layer (123) and an exclusion layer (126), wherein the electrically conductive first layer (123) is configured between the biocompatible material (120) and the exclusion layer (126).

## Patentansprüche

1. Biosensor (10) umfassend eine Biosensoreinheit (100) mit einer Elektrode (110), wobei die Elektrode (110) stabförmig ist, wobei die Elektrode (110) ferner einen Träger (120) mit einer elektrisch leitfähigen ersten Schicht (123) und einer Ausschlussschicht (126) umfasst, wobei die elektrisch leitfähige erste Schicht (123) zwischen dem Träger (120) und der Ausschlussschicht (126) konfiguriert ist, wobei die Elektrode (110) eine Nadelform mit einer nadelförmigen Spitze umfasst, wobei der Träger (120) ein Material umfasst, das ausgewählt ist aus der Gruppe bestehend aus Edelstahl, einem Carbid, Titan, Vanadium, Tantal und Wolframmetall, wobei die elektrisch leitfähige erste Schicht (123) einen oder mehrere von Gold, Silber, Silberoxid, Platin und Kohlenstoff umfasst und wobei die Biosensoreinheit (100) ferner ein Enzym (130) umfasst, das an der Elektrode (110) befestigt ist.

2. Biosensor (10) nach einem der vorhergehenden Ansprüche, wobei der Träger (120) ein Material mit einer Druckfestigkeit von mindestens 20.000 kPa umfasst.

3. Biosensor (10) nach einem der vorhergehenden Ansprüche, wobei der Träger (120) ein oder mehrere biokompatible Materialien umfasst.

4. Biosensor (10) nach einem der vorhergehenden Ansprüche, wobei die elektrisch leitfähige erste Schicht (123) eine Schichtdicke im Bereich von 3 nm bis 100 µm aufweist und wobei die Ausschlussschicht (126) eine Schichtdicke im Bereich von 1 nm bis 50 µm aufweist.

5. Biosensor (10) nach einem der vorhergehenden Ansprüche, wobei die Ausschlussschicht (126) für einen oder mehrere von H₂O_{2,} O₂ und NO durchlässig ist oder eine Elektronenübertragung (ET) ermöglichen kann und wobei die Ausschlussschicht (126) für einen oder mehrere von Ascorbat, 3,4-Dihydroxyphenylessigsäure (DOPAC), Dopamin und Harnsäure undurchlässig ist.

6. Biosensor (10) nach einem der vorhergehenden Ansprüche, wobei die Ausschlussschicht (126) eines oder mehrere von sulfoniertem tetrafluorethylenbasiertem Fluorpolymer-Copolymer, N,N'-Di-[(1-naphthyl)-N,N'-diphenyl]-1,1'-biphenyl)-4,4'-diamin (NPD) und p-Phenylendiamin (PPD) umfasst.

7. Biosensor (10) nach einem der vorhergehenden Ansprüche, ferner umfassend eine elektrisch leitfähige zweite Schicht (122), die zwischen dem Träger (120) und der elektrisch leitfähigen ersten Schicht (123) konfiguriert ist.

8. Biosensor (10) nach einem der vorhergehenden Ansprüche, wobei die Elektrode (110) einen Durchmesser von 4 mm oder weniger aufweist.

9. Biosensor (10) nach einem der vorhergehenden Ansprüche, wobei die Elektrode (110) eine Länge aufweist, die ausgewählt ist aus dem Bereich von 0,1 mm bis 15 mm, oder wobei die Elektrode (110) eine Länge aufweist, die ausgewählt ist aus dem Bereich von 0,1 bis 200 µm.

10. Biosensor (10) nach einem der vorhergehenden Ansprüche, wobei das Enzym einen oder mehrere einer Acetylcholinesterase, Cholinoxidase, Alkoholoxidase, D-Aminosäureoxidase, L-Aminosäureoxidase, Ascorbatoxidase, Aspartatoxidase, Catalase, Cholesterolesterase, Cholesteroloxidase, Galactoseoxidase, Glucoseoxidase, L-Glutamatoxidase, GABase, Glutaminase, Glycerolkinase, Glycerol-3-phosphatoxidase, Glycerol-3-phosphatoxidase, Hexokinase, Meerrettichperoxidase, Lactatoxidase, Pyruvatoxidase und Lysinoxidase umfasst.

11. Biosensor (10) nach einem der vorhergehenden Ansprüche, wobei der Biosensor (10) als ein Biosensor der ersten, der zweiten oder der dritten Generation konfiguriert ist.

12. Biosensor (10) nach einem der vorhergehenden Ansprüche, ferner umfassend eine Schutzschicht (129), die zum Einschließen des Enzyms konfiguriert ist.

13. Biosensor (10) nach einem der vorhergehenden Ansprüche, ferner umfassend eine Referenzelektrode (140), wobei die Referenzelektrode eine Ag/AgCI-Elektrode umfasst.

14. Biosensor (10) nach einem der vorhergehenden Ansprüche, umfassend einen Vorverstärker (200) in funktioneller Verbindung mit der Biosensoreinheit (100) und mit der Biosensoreinheit (100) funktionell verbunden.

15. Biosensor (10) nach einem der vorhergehenden Ansprüche, wobei die Ausschlussschicht (126) für H₂O₂ durchlässig ist, wobei die Ausschlussschicht (126) Nafion umfasst, wobei der Träger (120) Metall oder eine Metalllegierung umfasst, die eine Scherkraft von mindestens 40 GPa aufweist, wobei die Elektrode (110) einen Durchmesser von 100 µm oder weniger aufweist, wobei der Biosensor (10) ferner eine elektrisch leitfähige zweite Schicht (122) umfasst, die zwischen dem Träger (120) und der elektrisch leitfähigen ersten Schicht (123) konfiguriert ist, wobei die elektrisch leitfähige zweite Schicht (122) Kupfer umfasst.

16. Biosensor (10) nach einem der vorhergehenden Ansprüche, der Außenabmessungen aufweist, die gleich oder kleiner als 20 mm sind.

17. Biosensor (10) nach einem der vorhergehenden Ansprüche, umfassend mindestens zwei Elektroden, wobei die Elektroden einen kürzesten Abstand von 80 µm oder weniger aufweisen, oder wobei die Elektroden einen kürzesten Abstand von mindestens 0,5 mm aufweisen.

18. Biosensor (10) nach einem der vorhergehenden Ansprüche, umfassend mindestens sechs Elektroden (110).

19. Abtastsystem (1000), umfassend den Biosensor (10) nach einem der vorhergehenden Ansprüche, wobei der Biosensor (10) mindestens zwei Elektroden umfasst, wobei das Abtastsystem (1000) ferner eine Quelle (300) von elektrischer Energie in funktioneller Verbindung mit den mindestens zwei Elektroden umfasst, und einen Detektor (400), der zum Messen eines elektrischen Signals zwischen mindestens zwei der mindestens zwei Elektroden konfiguriert ist.

20. Verfahren zum Herstellen einer Elektrode (110) für einen Biosensor (10) zum Abtasten eines endogenen Analyts nach einem der Ansprüche 1 bis 18, durch Konstruieren einer Sensoroberfläche auf einem biokompatiblen Material (12), das für invasive Eingriffe an Menschen und/oder Tieren bestimmt ist, wobei die Sensoroberfläche (13) mindestens eine elektrisch leitfähige erste Schicht (123) und eine Ausschlussschicht (126) umfasst, wobei die elektrisch leitfähige erste Schicht (123) zwischen dem biokompatiblen Material (12) und der Ausschlussschicht (126) konfiguriert ist.

## Revendications

1. Biocapteur (10) comprenant une unité de biocapteur (100) avec une électrode (110), dans lequel l'électrode (110) est en forme de tige, dans lequel l'électrode (110) comprend en outre un support (120) avec une première couche électroconductrice (123) et une couche d'exclusion (126), dans lequel la première couche électroconductrice (123) est configurée entre le support (120) et la couche d'exclusion (126), dans lequel l'électrode (110) comprend une forme d'aiguille avec une pointe de forme d'aiguille, dans lequel le support (120) comprend un matériau choisi dans le groupe constitué de l'acier inoxydable, d'un carbure, de titane, de vanadium, de tantale, et de tungstène métallique, dans lequel la première couche électroconductrice (123) comprend un ou plusieurs corps parmi l'or, l'argent, l'oxyde d'argent, le platine et le carbone, et dans lequel l'unité de biocapteur (100) comprend en outre un enzyme (130) fixé à l'électrode (110).

2. Biocapteur (10) selon l'une quelconque des revendications précédentes, dans lequel le support (120) comprend un matériau ayant une résistance à la compression d'au moins 20 000 kPa.

3. Biocapteur (10) selon l'une quelconque des revendications précédentes, dans lequel le support (120) comprend un ou plusieurs matériaux biocompatibles.

4. Biocapteur (10) selon l'une quelconque des revendications précédentes, dans lequel la première couche électroconductrice (123) a une épaisseur de couche dans la plage de 3 nm à 100 µm et dans lequel la couche d'exclusion (126) a une épaisseur de couche dans la plage de 1 nm à 50 µm.

5. Biocapteur (10) selon l'une quelconque des revendications précédentes, dans lequel la couche d'exclusion (126) est perméable pour un ou plusieurs de H₂O₂, O₂, et NO, ou peut faciliter un transfert d'électrons (ET) et dans lequel la couche d'exclusion (126) est imperméable à une ou plusieurs substances parmi un ascorbate, l'acide 3,4-dihydroxyphénylacétique (DOPAC), la dopamine et l'acide urique.

6. Biocapteur (10) selon l'une quelconque des revendications précédentes, dans lequel la couche d'exclusion (126) comprend un ou plusieurs parmi un copolymère de fluoropolymère à base de tétrafluoroéthylène sulfoné, une N,N'-di-[(1-naphtyl)-N,N'-diphényl]-1,1'-biphényl)-4,4'-diamine (NPD), et la p-phénylènediamine (PPD).

7. Biocapteur (10) selon l'une quelconque des revendications précédentes, comprenant en outre une deuxième couche électroconductrice (122) configurée entre le support (120) et la première couche électroconductrice (123).

8. Biocapteur (10) selon l'une quelconque des revendications précédentes, dans lequel l'électrode (110) a un diamètre de 4 mm ou moins.

9. Biocapteur (10) selon l'une quelconque des revendications précédentes, dans lequel l'électrode (110) a une longueur choisie dans la plage de 0,1 mm à 15 mm ou dans lequel l'électrode (110) a une longueur choisie dans la plage de 0,1 à 200 µm.

10. Biocapteur (10) selon l'une quelconque des revendications précédentes, dans lequel l'enzyme comprend une ou plusieurs parmi l'acétylcholinestérase, l'oxydase de choline, l'oxydase d'alcool, l'oxydase d'acide D-aminé, l'oxydase d'acide L-aminé, l'oxydase d'ascorbate, l'oxydase d'aspartate, la catalase, l'estérase de cholestérol, l'oxydase de cholestérol, l'oxydase de galactose, l'oxydase de glucose, l'oxydase de L-glutamate, la GABase, la glutaminase, la glycérol kinase, l'oxydase de glycérol-3-phosphate, l'oxydase de glycérol-3-phosphate, l'hexokinase, la peroxydase de raifort, l'oxydase de lactate, l'oxydase de pyruvate et l'oxydase de lysine.

11. Biocapteur (10) selon l'une quelconque des revendications précédentes, dans lequel le biocapteur (10) est configuré sous la forme d'un biocapteur de première, deuxième ou troisième génération.

12. Biocapteur (10) selon l'une quelconque des revendications précédentes, comprenant en outre une couche protectrice (129) configurée pour enserrer l'enzyme.

13. Biocapteur (10) selon l'une quelconque des revendications précédentes, comprenant en outre une électrode de référence (140), dans lequel l'électrode de référence comprend une électrode d'Ag/AgCl.

14. Biocapteur (10) selon l'une quelconque des revendications précédentes, comprenant un préamplificateur (200) en liaison fonctionnelle avec l'unité de biocapteur (100) et fonctionnellement connecté à ladite unité de biocapteur (100).

15. Biocapteur (10) selon l'une quelconque des revendications précédentes, dans lequel la couche d'exclusion (126) est perméable pour H₂O₂ dans lequel la couche d'exclusion (126) comprend du Nafion, dans lequel le support (120) comprend un métal ou un alliage métallique ayant une résistance au cisaillement d'au moins 40 GPa, dans lequel l'électrode (110) a un diamètre de 100 µm ou moins, le biocapteur (10) comprenant en outre une deuxième couche électroconductrice (122) configurée entre le support (120) et la première couche électroconductrice (123), dans lequel la deuxième couche électroconductrice (122) comprend du cuivre.

16. Biocapteur (10) selon l'une quelconque des revendications précédentes, ayant des dimensions externes qui sont égales ou inférieures à 20 mm.

17. Biocapteur (10) selon l'une quelconque des revendications précédentes, comprenant au moins deux électrodes, dans lequel les électrodes ont une distance la plus courte de 80 µm ou moins, ou dans lequel les électrodes ont une distance la plus courte d'au moins 0,5 mm.

18. Biocapteur (10) selon l'une quelconque des revendications précédentes, comprenant au moins six électrodes (110).

19. Système de détection (1000) comprenant le biocapteur (10) selon l'une quelconque des revendications précédentes, dans lequel le biocapteur (10) comprend au moins deux électrodes, dans lequel le système de détection (1000) comprend en outre une source (300) d'énergie électrique en connexion fonctionnelle avec les au moins deux électrodes, et un détecteur (400) configuré pour mesurer un signal électrique entre au moins deux des au moins deux électrodes.

20. Procédé de fabrication d'une électrode (110) pour un biocapteur (10) afin de détecter un analyte endogène comme défini dans l'une quelconque des revendications 1 à 18, en construisant une surface de capteur sur un matériau biocompatible (12) destiné à des procédures invasives chez des humains et/ou des animaux, dans lequel la surface du capteur (13) comprend au moins une première couche électroconductrice (123) et une couche d'exclusion (126), dans lequel la première couche électroconductrice (123) est configurée entre le matériau biocompatible (12) et la couche d'exclusion (126).
